# EUROPEAN PATENT APPLICATION

(11) **EP 2 446 828 A1**
(43) Date of publication of application: **02.05.2012**
(21) Application number: 11187021.8
(22) Date of filing: 28.10.2011
(51) Int. Cl.: A61B 10/00

(54) **Sample collector with adequacy sensor**

(30) Priority: 29.10.2010 US 408450 P
(71) Applicant: Alere San Diego, Inc., San Diego, CA 92121 (US)
(72) Inventor: Cogan, Lorraine, San Diego, California 92128 (US); Parsons, Mary Catherine, Chula Vista, California 91911 (US); Tarpey, Christopher, Los Angeles, California 90035 (US); Bautista, Lorraine, Chula Vista, California 91913 (US); Bouliane, Martin, Carlsbad, California 92011 (US); Zeis, John, San Marcos, California 92078 (US); Vorres, Steve, San Marcos, California 92078 (US)
(74) Representative: Lee, Nicholas John

(57) **Abstract**

The present invention provides a sample collection device for determining that an adequate amount of sample is collected, the sample collection device including a sample collection member for collecting a fluid sample and at least one electrode, the at least one electrode being in liquid communication with the sample collection member and including a processor. In one embodiment, the sample collection member includes a groove structure, wherein at least one electrode is located on each side of the groove.

## Description

### BACKGROUND OF THE INVENTION

### FIELD OF THE INVENTION

The invention relates to methods and devices for indicating that an adequate amount of sample has been collected by a sample collection device.

### BACKGROUND INFORMATION

Sample collection devices are often used to collect a fluid sample, for example a saliva sample, for performing an assay. Examples of sample collection devices include swabs or absorbent materials which are compressible when wet by a fluid sample. When absorbent materials are used, the fluid sample needs to be removed from the absorbent sample collection material to be applied to an assay device. In some assay devices, such as the devices described in U.S. Patent No. 6,489,172, the sample collection devices include a sample collection member connected to a handle. The sample collection device is placed in the mouth of a subject and then placed into an assay device. The sample collection member becomes compressible due to the addition of saliva, allowing sample to be extracted from the sample collection member when compressed in the assay device. The collection device allows samples to be collected quickly, but there is no indication that an adequate amount of sample has been collected by the sample collection member.

In some sample collection devices, such as those described in U.S. Patent No. 7,114,403, a sample collection device include a sample collection member connected to a handle. The sample collection device is placed in the mouth of a subject, and then placed in an extraction device which can collect and store the liquid sample until it is added to an assay device. The extraction device also includes an aperture through which sample can be applied to the assay device.

In other sample collection and testing devices, such as those described in U.S. Patent No. 6,998,273, the sample collection device and the assay device are provided as a single device. The device contains a movable barrier between the sample collection area and the detection area, which allows for a certain amount of sample to be collected before the sample is applied to the assay device.

Traditional sample collection devices do not provide any indication that an adequate amount of sample has been collected by the device. To obtain an accurate assay result, an adequate amount of sample applied to the assay device. Without any indication of the adequacy of the amount of sample collected, a user may collect an inadequate amount of sample to apply to the assay device, which may result in an inaccurate assay result.

### SUMMARY OF THE INVENTION

The present invention includes in one embodiment a device for indicating the collection of an adequate amount of fluid. The device includes a handle, an sample collection member located at one end of the handle, at least one electrode in fluid communication with the sample collection member, and at least one signal indicator connected to the at least one electrode, the signal indicator indicating that the adequate amount of fluid has been absorbed by the sample collection member.

In embodiments, the sample collection member includes a groove structure. The at least one electrode may be located on a side of the groove structure. The at least one electrode further comprises a processor.

In embodiments, the signal indicator comprises at least one light-emitting diode. The device may also include an adhesive disk located between the sample collection member and the electrode.

The present invention includes a method of indicating the collection of an adequate amount of fluid may include steps of placing a collection device in the mouth of a user, and observing the signal indicator to determine when the adequate amount of fluid has been absorbed the sample collection member.

### BRIEF DESCRIPTION OF THE DRAWINGS

Figure 1 is a drawing of an embodiment of the sample collection device.

Figure 2 is a drawing of an embodiment of the sample collection device.

Figure 3 is a drawing of an alternative embodiment of the sample collection device.

Figure 4 is a drawing of a test strip.

Figure 5 is a top view of an alternative embodiment of the sample collection device.

Figure 6 is an interior view of the sample collection device of Figure 5.

Figure 7 is a side interior view of the sample collection device of Figure 5.

Figure 8 is an exploded view of the sample collection device of Figure 5.

Figure 9 is a drawing of a chassis portion of the sample collection device of Figure 5.

Figure 10 is a drawing of an alternative embodiment of the sample collection device.

Figure 11 is an exploded view of the sample collection device of Figure 10.

Figure 12 is a side view of the sample collection device of Figure 10.

Figure 13 is an interior view of the sample collection device of Figure 10.

Figure 14 is a drawing of an alternative embodiment of the sample collection device.

Figure 15 is an exploded view of the sample collection device of Figure 14.

Figure 16 is a side view of the sample collection device of Figure 14.

Figure 17 is an interior view of the sample collection device of Figure 14.

Figure 18 is a drawing of an alternative embodiment of the sample collection device.

Figure 19 is an exploded view of the sample collection device of Figure 18.

Figure 20 is a side view of the sample collection device of Figure 18.

Figure 21 is an interior view of the sample collection device of Figure 18.

Figure 22 is a three-dimensional drawing of the device of Figure 18.

Figure 23 is a drawing of the circuitry of the sample collection device.

Figures 24 A-H are drawings of alternative embodiments of the groove structure of the sample collection device.

### DETAILED DESCRIPTION OF THE INVENTION

The present invention includes devices and methods for the collection of fluid samples, for example a saliva sample, which indicate that an adequate amount of sample has been collected by the sample collection device.

In one embodiment, the invention includes a sample collection device, including a sample collection member for the absorption of a fluid sample, and electronic components for indicating that a sufficient amount of sample has been collected by the sample collection device. In embodiments, the sample is a fluid sample, for example, saliva, urine, blood, or other bodily fluids. The electronic components may include at least one electrode, a processor, and an signal indicator. The at least one electrode can be any conductive electrode conductor. In one embodiment, the device includes one electrode.

In another embodiment, the device includes two or more electrodes which are located at the same depth and distributed evenly across the cross-section of the sample collection device.

In another embodiment, the device includes three or more electrodes which are evenly distributed throughout the cross-section of the sample collection device to allow the electrodes to detect the collected fluid sample equally. In embodiments, the distance between the electrodes is 1-10 mm, preferably 2-5 mm. In embodiments, the depth of electrolyte into the chassis is 1-10 mm, preferably for 1-4 mm.

In embodiments, the sample collection member includes a groove structure. The at least one electrode can be located on a side of a groove. In embodiments, the device contains two electrodes which are located on each side of the groove.

In embodiments, the electronic components include at least one signal indicator. For example, the signal indicator may be any visible light or sound. In embodiments, the signal indicator is comprised of at least one light emitting diode. When an adequate amount of sample contacts the at least one electrode, the signal indicator indicates to a user that the proper amount of sample has been collected. In embodiments, the signal indicator is an audible signal.

In embodiments, the sample collection device includes a handle. The sample collection member is connected to one end of the handle, for example, through a chassis, or through an adhesive disk or ring. The handle may contain a window for viewing a signal indicator. The electronic components, such as wires and the at least one electrodes, may be located in the handle, and may contact the sample collection member at a junction between the sample collection member and the handle.

In embodiments, the sample collection device includes cavity at the junction between the sample collection member and the handle. The signal indicator, for example at least one light-emitting diode, may be located in the cavity. The cavity and/or signal indicator may be covered by a transparent material. The sample collection device may also comprise a hollow rod, the hollow rod in contact with the cavity and located in the handle of the sample collection device. The rod may contain the electronic components of the device, for example, wires and a processor. The rod may include a rod channel.

In embodiments, the device includes a chassis, the chassis including at least one aperture through which the at least electrode may contact the sample collection member. The at least one electrode is in contact with the cavity and the chassis. The chassis may be connected to the handle, for example by adhesive bonding, for example an adhesive ring or disk or adhesive film.

In embodiments, the sample collection device includes an assay device. The assay device may be located, for example, in the handle of the device. In another embodiment, the assay device is in fluid communication with the sample collection member. In another embodiment, the sample is applied to a separate assay device after the sample is collected by the sample collection member.

### Detection

Testing may be performed to determine the presence, absence or level of at least one substance or material, such as, but not limited to, chemical substances, organic compounds, inorganic compounds, metabolic products, drugs or drug metabolites, organic tissues or organisms, metabolites, nucleic acids, protein or polymer. A test may be performed to determine multiple substances or materials. Further, tests may be performed for immune detection, chemical detection, or enzyme detection.

### Sample

The present devices and methods can be used to collect samples for analysis in diagnostic and/or laboratory tests to determine the presence, absence, or amount of a material or substance. The sample can be a fluid sample, for example, a liquid sample, i.e. blood, plasma, serum, urine, saliva and various secretions, and can also include solid and semi-solid samples after the sample is pre-treated to form a liquid solution. Samples collected can be used for immune detection, chemical detection, or enzyme detection methods to detect the presence or amount of analyte.

### Analyte

The present devices and methods can be used to detect an analyte. The analyte can be detected in any liquid or liquefied sample, such as urine, saliva, saliva, blood, plasma, or serum. The devices and methods of the present invention may be used to detect, for example, analytes including but not limited human chorionic gonadotropin (HCG), luteinizing hormone (LH), follicle stimulating hormone (FSH), hepatitis C virus (HCV), hepatitis B virus (HBV), hepatitis B surface antigen, HIV, or any drug of abuse. The term "drug of abuse" ("DOA") is used to describe drugs that are used for non-medical purposes. These drugs are absorbed by the body and broken down into different small molecules, which are present in blood, urine, saliva, sweat and other bodily fluids.

### Sample collection member

In embodiments, the sample collection device includes a sample collection member. The sample collection member may absorb and store the sample. The sample collection member may be constituted of a material selected from, for example, polyvinyl alcohol (PVA), foam, resin, sodium polyacrylate cross-linked material, acrylic acid - ethylene acid copolymer, acrylonitrile polymer unsaponifiable matters, vinyl alcohol, sponge, filter paper, glass fiber, nonwoven, cellulose acetate, or a combination of one or several of these materials. In a preferred embodiment, the sample collection member is made of polyvinyl alcohol foam. The sample collection member can be any shape, including but not limited to cylinder shaped, rectangular, cone-shaped and any other suitable shape.

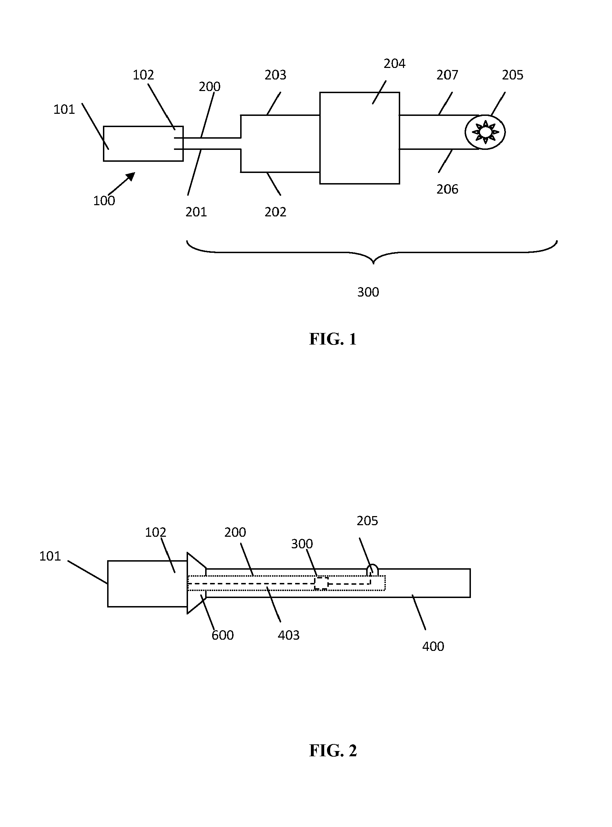

In the embodiment shown in FIG. 1, sample collection member **100 has** a first end **101** and a second end **102.** The sample collection member **100** can be inserted into the mouth of a user to obtain a sample, such as a saliva sample. The second end **102** may be in direct and/or fluid communication with electrodes **200, 201.** Electrodes **200, 201** are connected to processor **204** by direct or indirect contact with conductors or connections, for example wires **202, 203.** Processor **204** is connected to signal indicator **205** by wires **206, 207.** When sample is collected by sample collecting member **100,** electrodes **200, 201** are contacted by the sample and generate an electronic signal when an adequate amount of sample has been collected. The processor **204** receives this signal, and the signal indicator **205** indicates to the user that an adequate amount of sample has been collected and that the user can cease sample collection.

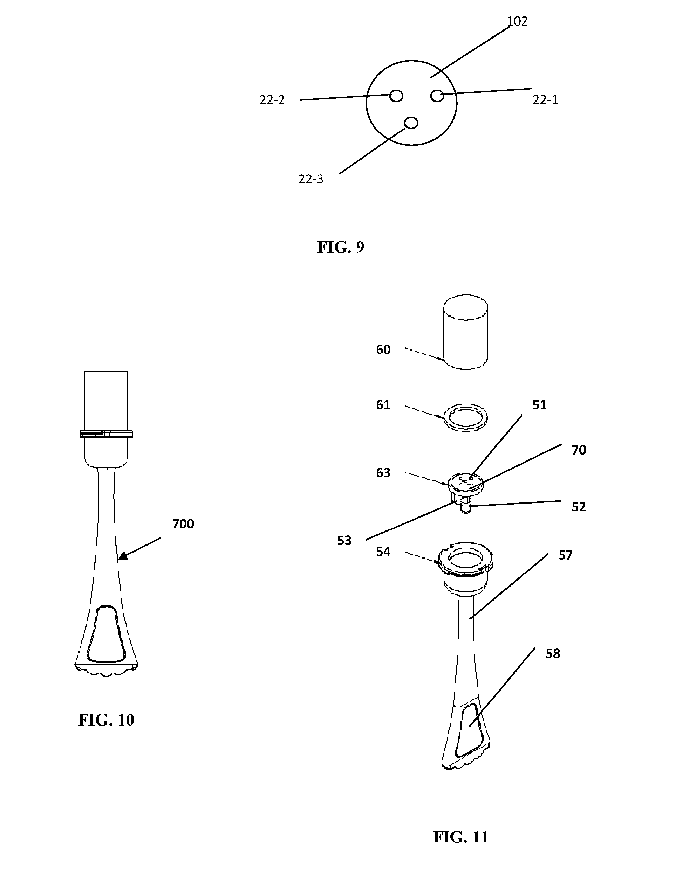

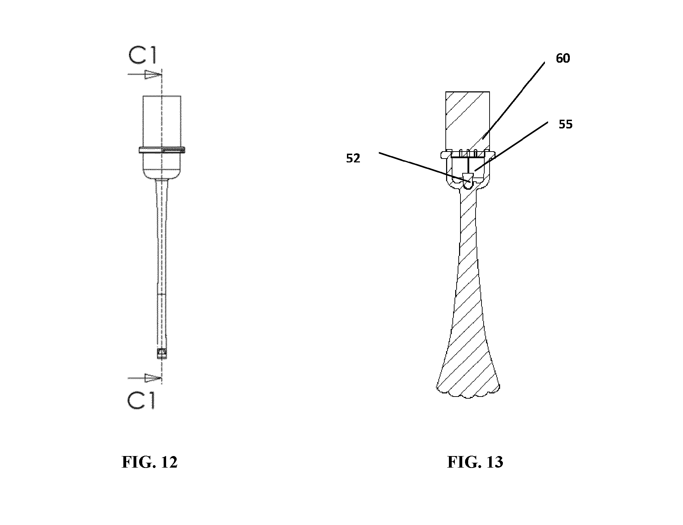

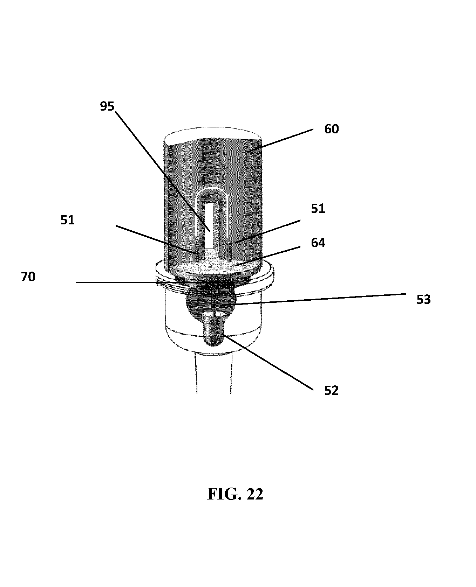

In the embodiment shown in FIGS. 10-13, sample collection member **60** is connected to a first end of the handle **57** using an adhesive ring or film **61.** The first end of the handle portion **57** of the device also includes chamber **54** and chamber cavity **55.** The handle also includes a second end **58** for gripping the handle. Conductive electrodes **51** are in fluid communication with sample collection member **60.** Electrodes **51** are in direct or indirect communication with at least one battery **53** and at least signal generator, for example a light-emitting diode **52.** These components form a current loop when the two or more electrodes **51** indicate that the sample collection member **60** has collected an adequate amount of sample, causing the light-emitting diode **52** to emit light. Electrodes **51** can also be arranged in a chassis **70,** the electrodes **51** protruding through the surface of chassis **70** to contact sample collection member **60.** Chassis **70** can also integrate light-emitting diode 52, battery **53,** or other components. Chassis **70** components are shown in more detail in **FIG. 22****.**

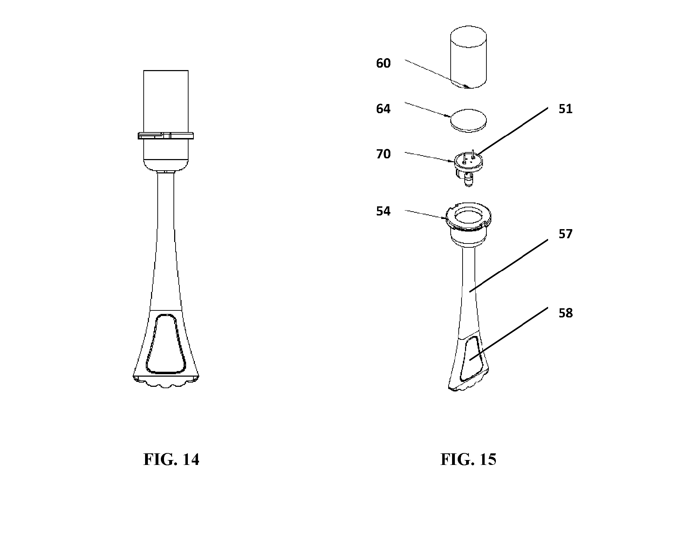

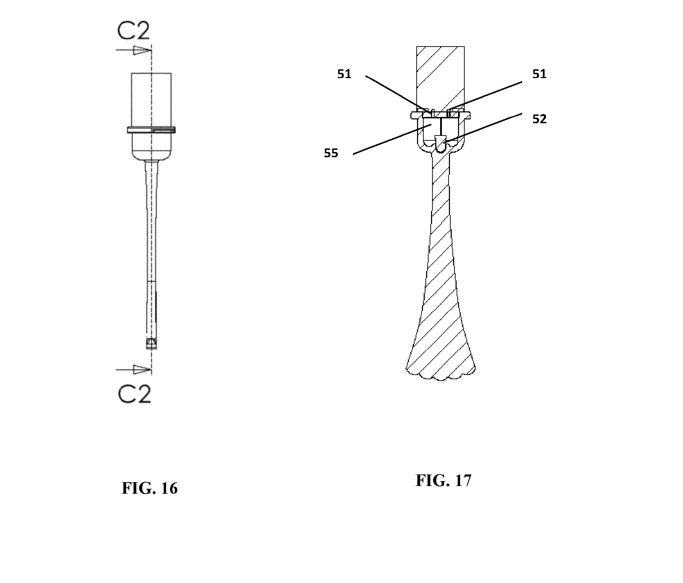

Another embodiment is shown in FIGS. 14-17. In this embodiment, sample collection member **60** is bonded to chassis **70** and the handle by an adhesive disc **64.** Electrodes **51** protrude through adhesive disc **64** to contact the sample collection member **60.**

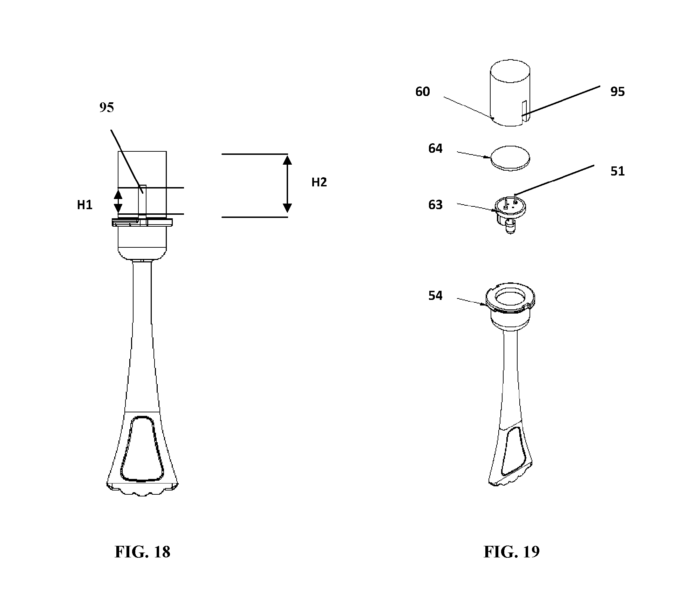

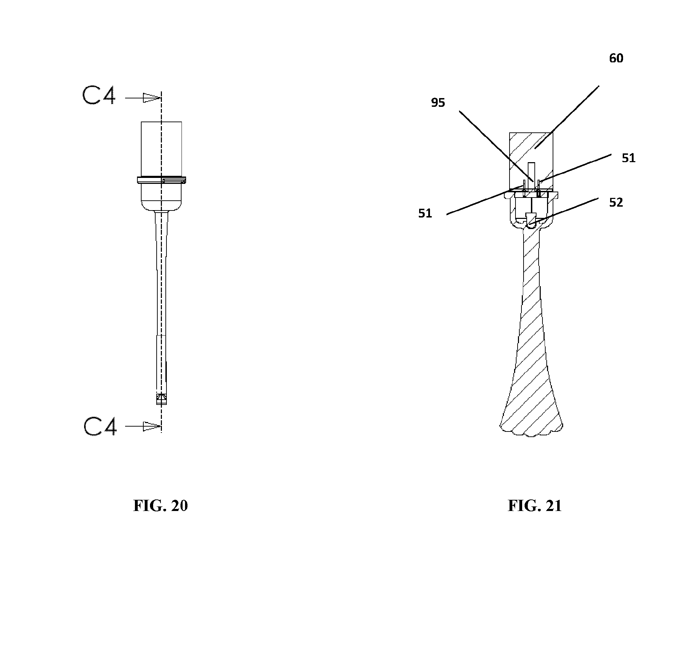

FIGS. 18-22 show another embodiment, wherein sample collection member **60** includes a groove structure **95.** As shown in FIG. 22, electrodes **51** are located on both sides of groove **95.** When the sample collection member **60** collects an adequate amount of sample, the electrodes **51** indicate that enough liquid has been collected, and that the signal indicator should be turned on. The groove **95** in the sample collection member **60,** including the structure on both sides of the groove, can be filled with sample to at least to the electrode conduction portion.

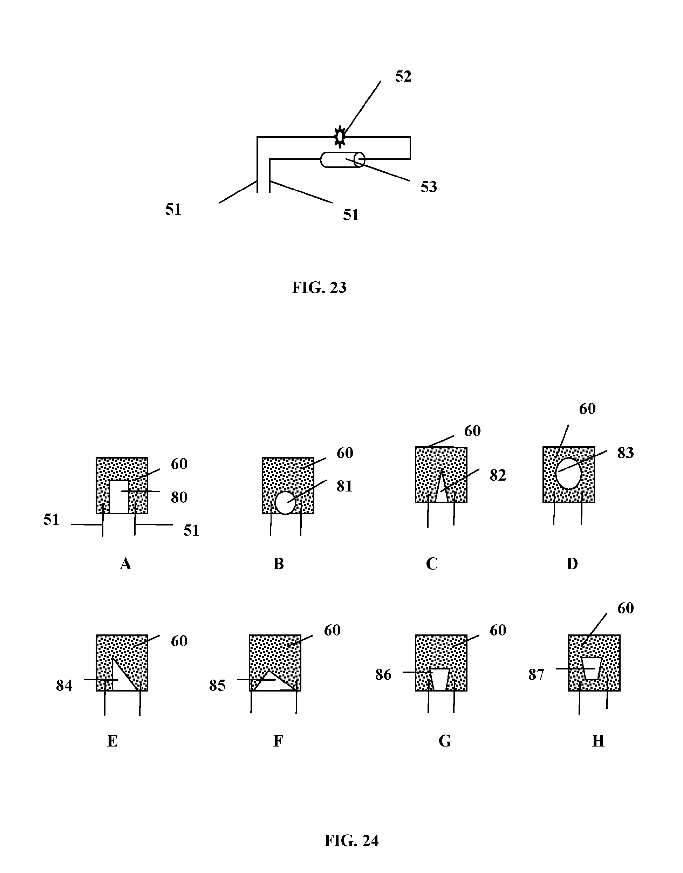

In embodiments, the groove **95** can run through the entire sample collection member **60,** and divides the sample collection member **60** equally into two parts. Two electrodes **51** are located in the groove on the left side and the right side. In a preferred embodiment, groove **95** does not completely separate the sample collection member into two parts; preferably, the height of the groove **95** is at least a height **H1** and is less than a height **H2** as shown in FIG. 18. By way of the groove **95,** the sample collection member **60** can absorb sample and avoid the possibility of conduction in the electrodes **51** before an adequate amount of sample is collected. The groove can be any shape, for example, a channel, cylindrical, rectangular, trapezoidal and other shapes, for example as shown in FIG. 23.

As shown in FIGS. 10-22, the handle of the sample collection device includes a channel, one end of the channel connected to cavity **54,** the other end of the channel connected to the second end of the handle **58.** When the light-emitting diode is lit, light can pass through the channel to the second end of the handle **58,** which can be made of a transparent material such as transparent plastic, glass, etc. so that a user can see the light and cease sample collection.

### Handle

As shown in FIGS. 1 and 2, sample collection member **100** is connected to handle **400.** Handle **400** may include windows at the end of the opening of cavity **403.** The windows may be shaped and sized to allow for the signal indicator **205** to be observed through the window. Handle **400** can also include chassis **600,** inner cavity **403** of the chassis, and adhesive to attach sample collection member **100** to handle **400.** In a preferred embodiment, the electronic components **300** are located in the handle **400** within the inner cavity **403.**

Electrodes **200, 201** are located in the cavity **403,** the electrodes **200, 201** in contact with both chassis **600** and the second end **102** of sample collection member **100.** A signal indicator **205** receives an indicator signal sent through the device. The signal indicator **205** may be at least one light-emitting diode, and can be located on the handle in a window. When sample is collected and the sample reaches the second end **102** of the sample collection member **100,** the sample contacts two separate electrodes **200, 201,** and the electrodes produce an signal which is send to the processor **204** and then to the signal indicator **205,** indicating that an adequate amount of sample has been collected.

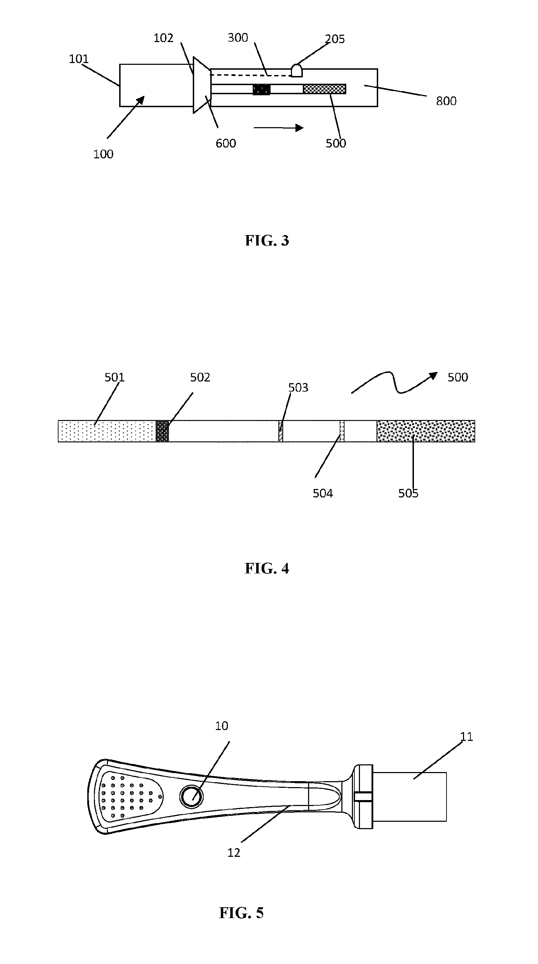

In another embodiment, the collection device includes a test element. As shown in FIGS. 3-4, a test element **500** may be located within the housing of a test device **800.** The test device **800** further comprises a sample collection member **100,** a chassis **600,** and a signal indicator **205.** The test element **500** is in fluid communication with the sample collection member, such that sample can flow directly to the test element **500.** Test element **500** includes a sample receiving zone **501,** a reagent zone **502,** a detection zone **503,** a control zone **504,** and an absorption area **505.** The sample collection member **100** and the sample receiving zone **501** are in fluid communication. Sample flows from the sample collection member **100** to the sample receiving zone **501** and along the test element **500.** The second end **102** of the sample collection member **100** may be in direct or indirect contact with the sample receiving zone **501.** These components may be in indirect contact through a material, such as filter paper. The electronic components of the device may also be located in the housing. The signal indicator may be located in a window of the housing.

### Electronic Alert Device

The sample collection device can include a sample collection member in contact with at least a portion of at least one electrode, and a processor connected to electronic components. These electronic components can be connected through a conductor or semiconductor, allowing electronic signals to be transmitted between the various components. The processor can be programmed to control the signal processing and response. For example, when sample is collected in the sample collection member, the sample contacts the electrodes produce an electronic signal. The electronic signal is received by the processor, and is sent to another electronic component, for example, a signal indicator to produce a signal, such as light or sound, which indicates that the amount of sample collected is sufficient, or has reached a pre-set amount. In a preferred embodiment, the sample collection member is in contact with at least two electrodes. In the initial stage of use, a microvoltage is applied between the two electrodes. When the two electrodes are contacted by sample to constitute a current path between the electrodes, a current path is generated by the processor after receiving an electronic signal from the electrodes. The electronic signal is sent to the signal indicator, indicating that the amount of sample collected is sufficient. The signal from the electronic components can travel through a conductor, such as wires connected to the processor. A sufficient or adequate amount of sample refers to the amount of sample required meet pre-set requirements, such as minimum volume or weight requirements set by a laboratory or a diagnostic test manufacturer, such as 1 ml, 5 ml or 1-10 ml. The electrodes can be configured within the sample collection member to achieve a requirement for indicating a certain level of sample. As shown in FIG. 1, two electrodes **200, 201** are in contact with the sample collection member **100.** When sample contacts the two electrodes, the signal conducted between two electrodes is sent to the processor and the signal indicator. If the two electrodes are located in the middle of the sample collection member, the signal will be produced when the sample collection member is half-filled with sample. In this manner, the depth of the electrodes can be adjusted to reach a pre-set amount of volume or weight of sample.

### Signal indicator

Any type of signal indicator can be used to indicate that a sufficient amount of sample has been collected, such as visual signals, audible signals, or any other signal that can be understood by a user. The signal indicator may include at least one light-emitting diode, or a speaker for emitting an audible signal. Identification signal can also be a variety of other forms, i.e. color changes, sound reminders, or light reminders. For example, a specific sound, such as an alarm sound, can be used to indicate to a user that a sufficient amount of sample has been collected. Visual signals can also be used in various ways, for example, by a change in color or brightness of light-emitting diodes.

### Detection device

The collected sample can be applied to a test element **500** of a detection device, as shown in FIGS. 3-4, to determine the presence or amount of at least one analyte. Sample is applied to the sample receiving zone **501.** The sample flows to a reagent zone **502,** a detection zone **503,** a control zone **504,** and an absorption area **505.** The sample collection member **100** and the sample receiving zone **501** are in fluid communication. The sample collection device may contain a detection device, wherein sample flows from the sample collection member **100** to the sample receiving zone **501** and along the test element **500.** The detection device may also be a separate assay device, to which sample is applied after it is collected by the sample collection device.

### Test components

Sample may be applied to a test strip, i.e. a lateral flow test strip, which can detect one or multiple analytes. Various test components can be used together to detect analyte. Any form of test strip may be used to analyze the sample for a particular analyte, i.e. drugs, drug metabolites, or other markers. The test strips may perform an immunoassay or chemical analysis. The assay can be a competitive assay or a sandwich assay. The test strip contains a sample receiving zone **501,** a reagent zone **502,** a detection zone **503,** a control zone **504,** and optionally an absorption area **505.** Sample is added to the sample receiving zone and flows downstream to the reagent zone by capillary action, where the sample contacts mobilisable labeled binding agents. The sample continues to flow into the detection zone. The detection zone contains immobilized reagents, such as reagents which specifically bind the analyte. When analyte is present in the sample, the analyte binds with the reagents in the detection zone. The test strip can be made of material such as a nitrocellulose membrane. The test strip can be adhered to a supporting material or a hard surface.

The following examples are intended to illustrate but not limit the invention.

### EXAMPLES

### EXAMPLE I

FIG. 1 shows a sample collection device including includes a sample collection member **100,** the sample collection member having a first end **101** and a second end **102,** the second end **102** in contact with electrodes **200, 201.** Electrodes **200, 201** are in contact with processor **204** through wires **202, 203.** Processor **204** is connected to signal indicator **205** through wires **206, 207.** When in use, a micro-voltage such as 1-20 microamps or 1-50 microamps, is applied to the electrodes, the signal indicator initially in the off state. When the first end **101** of the sample collection member **100** contacts a fluid sample, fluid is absorbed into the sample collection member **100** due to the adsorptive collective member and/or capillary action. As the sample collection member **100** is filled with sample, conduction electrodes **202, 203** produce an electronic signal to the processor **204,** which sends a signal to the signal indicator 205, which indicates that the sample collection member has collected sufficient sample for testing.

### EXAMPLE II

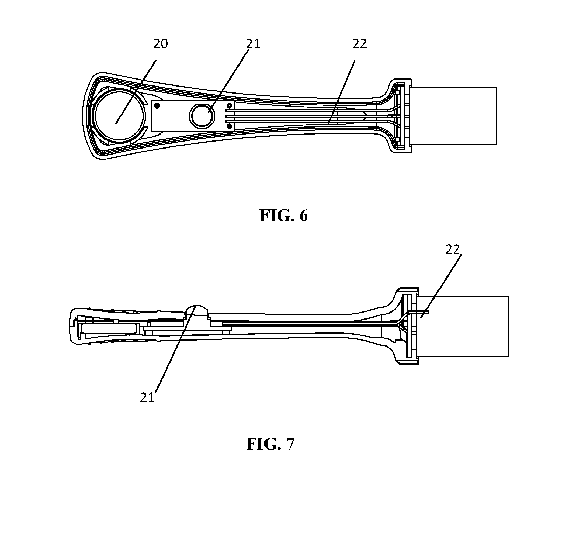

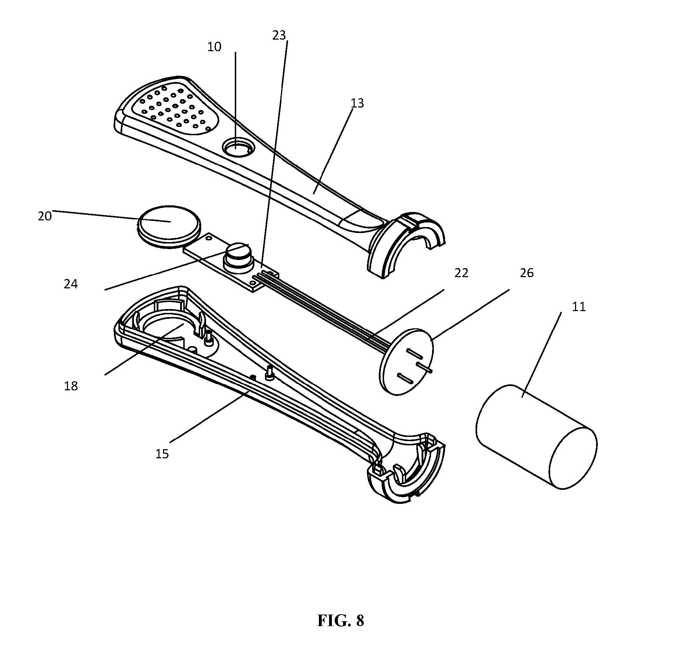

As shown in FIGS. 5-9, the sample collection device includes of a sample collection member **11** composed of a cylinder-shaped foam material, the sample collection member **11** connected at one end to a handle **12.** The handle **12** includes an upper part **13** and a lower part **15** which snap together to form the handle **12.** The handle **12** has a cavity **18** to accommodate the electronic components of the device. The device includes a battery **20** to provide power, a processor **23,** and a light-emitting diode **24** which is visible through window **10.** The processor is connected with the three electrodes **22-1, 22-2, 22-3.** One end of the electrodes protrudes through a chassis **26** to contact the sample collection member **10,** the chassis **26** distributing the electrodes evenly. The electrodes are inserted into the sample collection member to the same depth of 2mm. Before use, the light-emitting diode **24** is in an off state. A voltage of 10 microamps is applied to the electrodes. When the device is placed in the mouth, a saliva sample gradually fills sample collection member **11.** The saliva sample contacts the three electrodes **22-1, 22-2, 22-3.** When all three electrodes are contacted with saliva, the processor **23** generates an electric signal, which causes the light-emitting diode **24** to emit light through window **10.** This alerts the user that an adequate amount of sample has been collected.

### EXAMPLE III

As shown in FIGS. 14-17, the sample collection device includes sample collection member **60,** adhesive film **64,** chamber **54,** and chassis **70,** which contains two electrodes **51,** a light-emitting diode **52** and a battery. Two electrodes **51** are in contact with the sample collection member **60.** In this example, the distance between two electrodes is 2 mm, and the electrodes protruding about 4 mm from the chassis into the sample collection member.

Although the invention has been described with reference to the above example, it will be understood that modifications and variations are encompassed within the spirit and scope of the invention. Accordingly, the invention is limited only by the following claims.

### EXAMPLE IV

As shown in FIGS. 18-22, the sample collection device includes sample collection member **60,** adhesive film **64,** chamber **54,** and chassis **70,** which contains two electrodes **51,** a light-emitting diode **52** and a battery. Two electrodes **51** are in contact with the sample collection member **60.** In this example, the distance between two electrodes is 2 mm, and the electrodes protruding about 4 mm from the chassis into the sample collection member. The sample collection member includes a groove **95** of a height more than H1 and less than H2. Each of the electrodes **51** is located on one side of the groove.

### EXAMPLE V

As shown in FIGS. 10-13, the sample collection device includes sample collection member **60,** adhesive disc **61,** chamber **54,** and chassis **70,** which contains five electrodes **51,** a light-emitting diode **52** and a battery. Two electrodes **51** are in contact with the sample collection member **60.** In this example, the distance between two electrodes is 2 mm, and the electrodes protruding about 1 mm from the chassis into the sample collection member.

### EXAMPLE VI

Various configurations of the sample collection device were tested to determine the average volume of sample collected and the time required to collect the sample. Each configuration was tested three times. The average results for each configuration is listed in the table below.

| | Configuration 1 | Configuration 2 | Configuration 3 | Configuration 4 |
|---|---|---|---|---|
| **Time (seconds)** | **0.23** | **0.59** | **0.52** | **0.50** |
| **Volume (mL)** | **0.65** | **1.78** | **1.34** | **0.80** |

Although the invention has been described with reference to the above examples, it will be understood that modifications and variations are encompassed within the spirit and scope of the invention. Accordingly, the invention is limited only by the following claims.

## Claims

1. A device for indicating the collection of an adequate amount of fluid, the device comprising:
a handle;
a sample collection member located at one end of the handle;
at least one electrode in fluid communication with the sample collection member; and
at least one signal indicator connected to the at least one electrode, the signal indicator indicating that the adequate amount of fluid has been absorbed by the sample collection member.

2. The device of claim 1, wherein the sample collection member includes a groove structure.

3. The device of claim 2, wherein the at least one electrode is located on a side of the groove structure.

4. The device of claim 1, wherein the at least one electrode further comprises a processor.

5. The device of claim 1, wherein the signal indicator comprises at least one light-emitting diode.

6. The device of claim 1, the device further comprising an adhesive disk located between the sample collection member and the electrode.

7. A method of indicating the collection of an adequate amount of fluid, the method comprising:
placing a collection device in the mouth of a user, the collection device comprising:
a handle;
a sample collection member located at one end of the handle;
at least one electrode in fluid communication with the sample collection member;
at least one signal indicator connected to the electrode the signal indicator indicating that the adequate amount of fluid has been absorbed by the sample collection member; and
observing the signal indicator to determine when the adequate amount of fluid has been absorbed by the sample collection member.
